# EUROPEAN PATENT APPLICATION

(11) **EP 2 072 510 A1**
(43) Date of publication of application: **24.06.2009**
(21) Application number: 07382005.2
(22) Date of filing: 21.12.2007
(51) Int. Cl.: C07D 403/04, A61K 31/55, A61P 37/08

(54) **Crystalline form of azelastine**

(71) Applicant: ESTEVE QUIMICA, S.A., 08024 Barcelona (ES)
(72) Inventor: Fernaández Bleda, David, 08024, Barcelona (ES); Serres Mairal, Jordi, 08024, Barcelona (ES)
(74) Representative: Barlocci, Anna

(57) **Abstract**

Polymorph I of azelastine of formula (I), its preparation process which comprises the following steps: a) crystallizing azelastine from a solution of said compound in isobutylmethylketone; b) isolating the polymorph I of azelastine that appears in the prior step; and c) removing the organic solvent from the polymorph I of azelastine thus obtained, and its use as antihistamine.

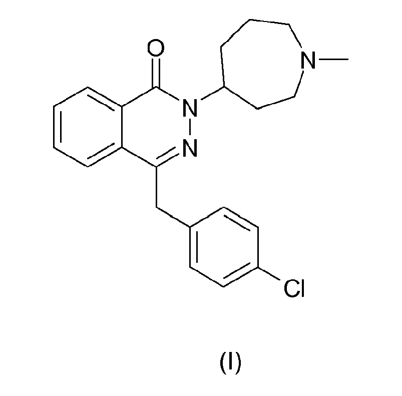

## Description

The present invention relates to a crystalline form of azelastine, a process for its preparation, its use as therapeuticallly active ingredient, and pharmaceutical compositions comprising it.

### BACKGROUND ART

Azelastine is the International Nonproprietary Name (INN) of 4-[(4-chlorophenyl)methyl]-2-hexahydro-1-methyl-1H-azepin-4-yl)-1(2H)-phtalazinone, and CAS No. 58581-89-8. For therapeutical purposes, it is preferably used in the form of hydrochloride salt. Azelastine and therapeutically acceptable salts thereof are well-known as antihistamine and mast cell stabilizer.

The structure of azelastine corresponds to formula (I):

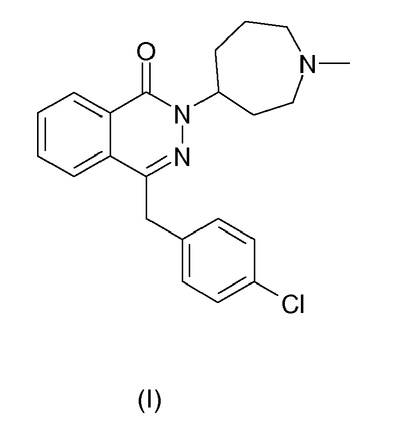

Different synthetic strategies for the preparation of azelastine and its salts are known. For instance, US3813384-A discloses certain substituted benzyl phtalazone derivatives including azelastine and its salts, methods for their preparation, and pharmaceutical compositions using these compounds. Several preparation processes of azelastine are reported in this document. Example 10 relates to the preparation of azelastine from p-chlorobenzylacetophenone-o-carboxylic acid by its reaction with hydrazine sulfate, followed by reaction of the compound obtained with 4-(p-chlorobenzyl)-1-(2H)-phthalazinone. According to this Example, the azelastine crude isolated is an oil, which is converted into its hydrochloride salt and purified by crystallization. Example 38 relates to the preparation of azelastine via methylation of 4-[(4-chlorophenyl)methyl]-2-hexahydro-1H-azepin-4-yl)-1 (2H)-phtalazinone with formaldehyde/formic acid. The compound is also isolated in form of its hydrochloride salt.

Unlike the known azelastine which is an oil, the known salts of azelastine are solids, which allow the manufacture of the salts on industrial scale and, in particular, their use for the preparation of pharmaceutical formulations. However, the preparation of a salt of azelastine involves an extra processing steps of preparing the salt and of purification, increasing the cost and the time to manufacture the compound. For instance, the hydrochloride salt results to be a fine powder that filters with difficulty, and which is also difficult to purifiy. Thus, the purification of azelastine still presents a number of difficulties.

Generally, the azelastine is the precursor compound for the preparation of the salt. Therefore, depending on the preparation process used for the preparation of the salt, the isolation step of the azelastine must also be done.

Two different crystalline forms of azelastine monohydrate are also known (cf. G.Sheffler et al., Arch. Pharm. 1988, vol. 321, pp. 205-208). This forms are obtained by crystallization of a mixture of ethanol/water.

Thus, there is still a need to find new solid forms of azelastine suitable to operate on industrial scale, either to be used directly as pharmaceutical active ingredient in a pharmaceutical formulation, or if desired, to be subsequently transformed into a pharmaceutically acceptable salt thereof.

Furthermore, the different solid forms of a pharmaceutically active ingredient can have different characteristics, and offer certain advantages, in methods of manufacture and also in pharmacology. Thus, the discovery of new solid forms can contribute to clear improvements in the efficiency of methods of production and/or improvements in the characteristics of the pharmaceutical formulations of the active ingredients, since some forms are more adequate for one type of formulation, and other forms for other different formulations.

### SUMMARY OF THE INVENTION

Inventors have found a crystalline form of azelastine with improved physical characteristics, which solves some of the problems previously mentioned. From our knowledge, it is the first crystalline form of anhydrous azelastine. This crystalline form is stable and easy to handle. Furthermore, it is not hygroscopic and does not trap solvent. The improved physical characteristics of said crystalline form compared with the azelastine described in the art, which is an oil, involve enhanced manufacturing capabilities and the provision of a compound suitable for use in the preparation of pharmaceutical formulations.

Therefore, it is a significant contribution to the art to provide azelastine in crystalline form and, particularly, to provide a crystalline form of azelastine with the ability to crystallize and the ability to filter easily, methods for its preparation, its use as a therapeutically active agent and pharmaceutical compositions comprising it.

The crystalline form of azelastine of the present invention is obtained with high yields and elevated richness, that is, with a purity greater than 99%.

Furthermore, its preparation process is consistently reproducible and robust, and, therefore, easily industrializable.

Thus, according to one aspect of the present invention, it is provided a crystalline form of azelastine of formula (I).

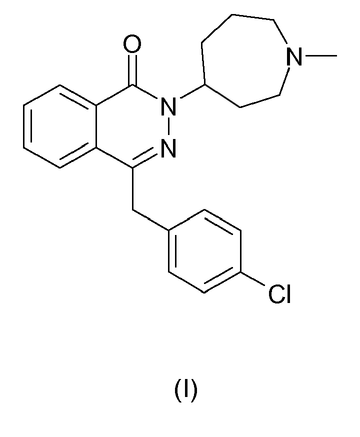

In another aspect of the present invention, it is provided a crystalline form of azelastine of formula (I) characterized by having an X-ray diffractogram that comprises characteristic peaks at approximately 7.1, 10.7, 12.1,13.5, 13.8, 16.8, 20.1, 20.8, 21.4 and 23.8 degrees 2 theta.

The new crystalline form of the present invention has been named polymorph I.

Hence, compared with azelastine obtained as an oil, the advantage of the crystalline form of the present invention is clear, since the product can be filtered and dried, thus easily isolated on industrial scale, an when needed, it could be recrystallized to improve the purity. Compared with the monohydrate form also known in the art, the polymorph I of azelastine shows an improved stability, since the hydration molecule of the monohydrate form is lost easily, with the drawback that then its composition is not well-defined.

A second aspect of the present invention relates to a process for the preparation of the polymorph I of azelastine as defined above, characterized by comprising the following steps: a) crystallizing azelastine from a solution of said compound in isobutylmethylketone; b) isolating the crystalline form of azelastine that appears in the prior step; and c) removing the organic solvent from the polymorph I of azelastine thus obtained.

Another aspect of the present invention relates to a pharmaceutical composition that comprises as active ingredient a therapeutically effective amount of a crystalline form of azelastine as defined above, in particular polymorph I, together with appropriate pharmaceutically acceptable excipients or carriers.

Finally, another aspect of the present invention relates to the use of a crystalline form of azelastine as defined above, in particular polymorph I, for the preparation of a medicament for the treatment and/or prophylaxis of allergic diseases. This aspect can also be formulated as polymorph I of azelastine as defined above for use in the treatment and/or prophylaxis of allergic diseases.

The invention is also related to a method of treatment and/or prophylaxis of a mammal, including a human, suffering from or being susceptible to allergic diseases, said method comprising the administration to said patient of a therapeutically effective amount of a crystalline form of azelastine, in particular polymorph I, together with pharmaceutically acceptable excipients or carriers.

### BRIEF DESCRIPTION OF THE DRAWINGS

FIG. 1 shows the X-ray diffraction spectrum of the polymorph I of azelastine of the present invention.
FIG. 2 shows the Differential Scanning Calorimetry (DSC) curve of the polymorph I of the azelastine of the present invention.
FIG. 3 shows the X-ray diffraction spectrum of the azelastine monohydrate.
FIG. 4 shows the Differential Scanning Calorimetry (DSC) curve of the azelastine monohydrate.

### DETAILED DESCRIPTION OF THE INVENTION

This new polymorph I of azelastine is characterized by exhibiting in the powder X-ray diffractogram a pattern of peaks, expressed in 2 theta units in degrees, 20 (°), which is shown in the following table:

| d (Å) | 2θ (°) | Relative intensity (%) |
|---|---|---|
| 7.16 | 12.3 | 59.6 |
| 10.70 | 8.2 | 55.3 |
| 11.19 | 7.9 | 2.0 |
| 12.06 | 7.3 | 10.6 |
| 13.51 | 6.5 | 21.2 |
| 13.85 | 6.4 | 47.2 |
| 16.82 | 5.3 | 13.2 |
| 18.43 | 4.8 | 11.1 |
| 18.75 | 4.7 | 7.6 |
| 19.27 | 4.6 | 1.6 |
| 20.10 | 4.4 | 71.0 |
| 20.78 | 4.3 | 100.0 |
| 21.43 | 4.1 | 13.4 |
| 21.76 | 4.1 | 5.5 |
| 22.51 | 3.9 | 4.5 |
| 23.37 | 3.8 | 3.9 |
| 23.82 | 3.7 | 10.6 |
| 25.20 | 3.5 | 8.0 |
| 25.65 | 3.5 | 4.2 |
| 26.20 | 3.4 | 5.6 |
| 27.21 | 3.3 | 1.6 |
| 27.91 | 3.2 | 8.5 |
| 28.49 | 3.1 | 5.5 |
| 29.00 | 3.1 | 5.8 |
| 29.40 | 3.0 | 6.7 |
| 30.19 | 3.0 | 1.3 |
| 30.61 | 2.9 | 1.2 |
| 31.36 | 2.8 | 5.5 |
| 31.65 | 2.8 | 4.4 |
| 32.52 | 2.8 | 0.8 |
| 33.11 | 2.7 | 1.9 |
| 35.15 | 2.5 | 1.8 |
| 35.50 | 2.5 | 0.9 |
| 37.62 | 2.4 | 2.3 |
| 39.17 | 2.3 | 1.2 |
| 39.83 | 2.6 | 3.4 |

X-ray diffractogram is obtained using the diffractometer PANalytical X'Pert PRO MPD diffractometer with goniometer theta/theta of 240 milimeters of radio, parallel optic with hybrid monochromator and transmission geometry and sample holders for capilars and spinner. Cu-Kα1 radiation (λ=1.5406 Ǻ). Power: 45 kV-40mA. Slip that determines the high of the beam of 0.19 milimeters. Soller windows of 0.02 radians in the incident and difracted beam. Detector X'Celerator with active length of 2.122°. Scannings of 2 θ from 2 to 50° 2 θ with passage size of 0.017° and measure of time of 300 segons per passage.

It is also characterized by Differential Scanning Calorimetry (DSC) showing a melting peak at approximately 113.1 °C.

DSC analysis were recorded in a Mettler Toledo DSC822e/500. Samples were heated, under air (60 mL/ min), from 25 to 250°C at a heating rate 10 °C/min.

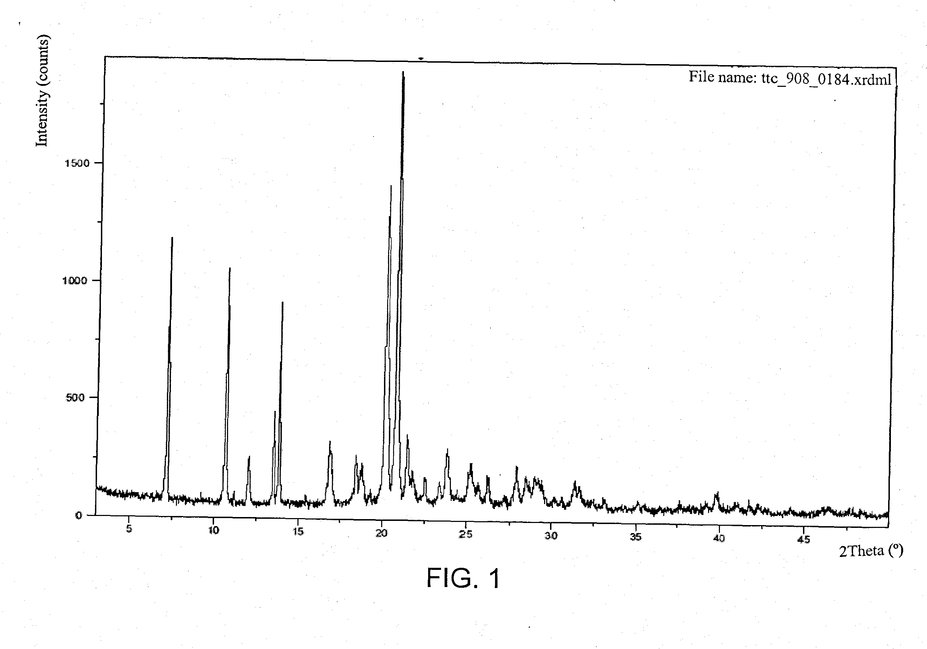

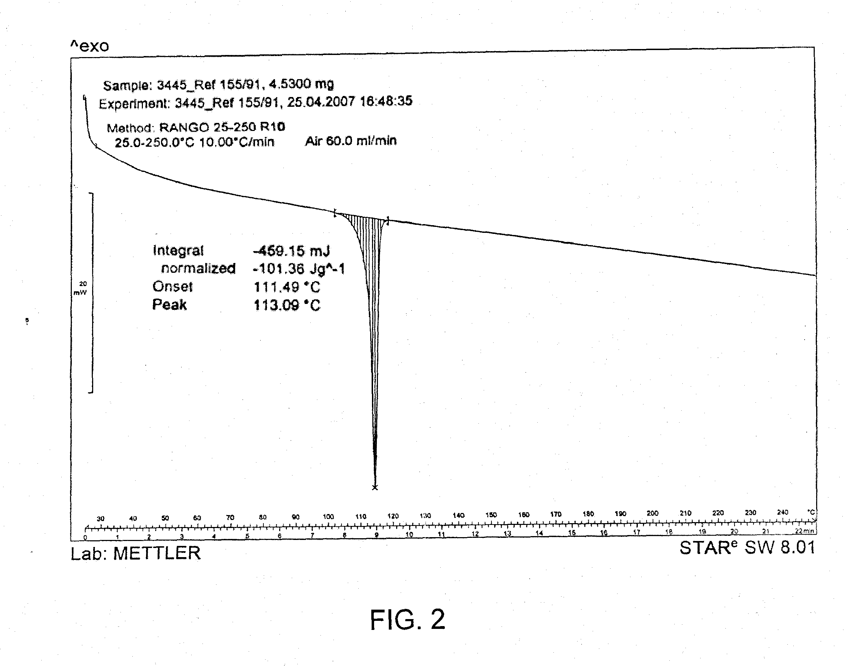

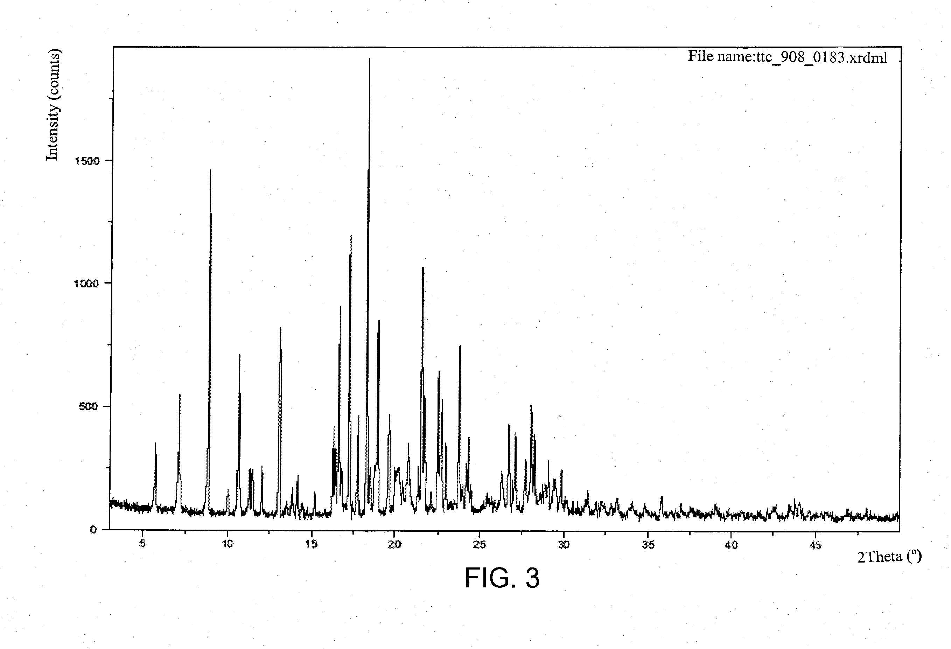

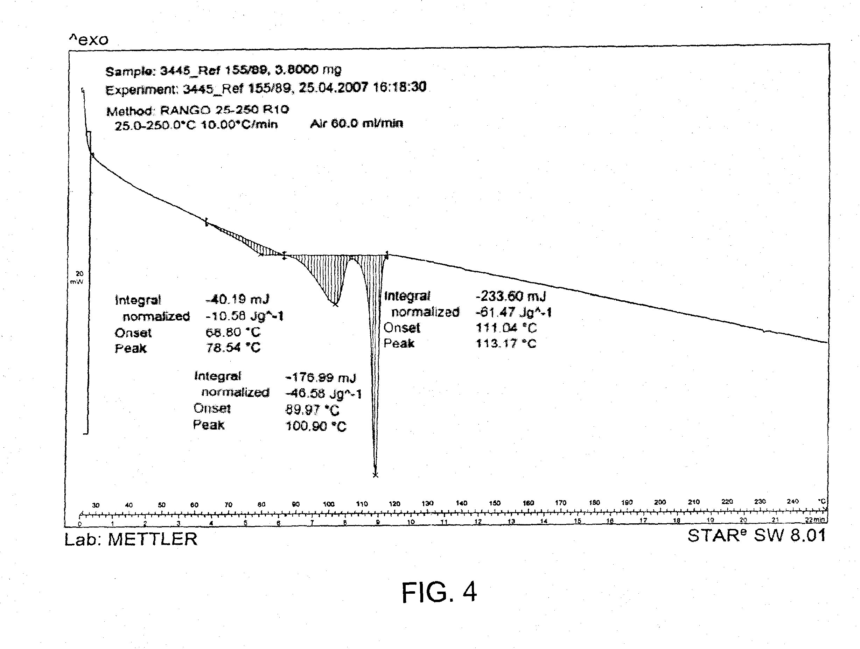

The anhydrous crystalline form of azelastine of the present invention, polymorph I, has an X-ray diffraction spectrum (FIG. 1) that differs from that of the monohydrate form (FIG. 3). It also differs in the DSC spectrum as can be seen by the comparison of FIG. 2 and FIG. 4.

This form is essentially free of solvent (including any organic solvent or water) and is free of solvation. By the expression "essentially free of solvent" it is understood that it complies with the relevant pharmaceutical specifications for the presence of solvents. By free solvent it is understood that it does not form part of the product's crystalline structure, and by solvation solvent it is understood that it is incorporated into the crystalline structure of the same.

As mentioned above, the polymorph I of azelastine of the present invention can be prepared by a process comprising the following steps: a) crystallizing azelastine from a solution of azelastine in isobutylmethylketone; b) isolating the polymorph I of azelastine that appears in the prior step; and c) removing the organic solvent from the polymorph I of azelastine thus obtained.

Generally, the azelastine is dissolved at a temperature near or at the boiling point of the solvent employed. Then, the hot solution is cooled causing the dissolved azelastine to crystallize out and the resulting suspension is kept at that temperature during some time.The resulting suspension can be perfectly stirred. The separation of the product from the supernatant solution can be done by a conventional method such as filtration. The solid filters readily, thereby assisting ease of preparation. The remaining solvent can be removed from the product by drying, optionally under vacuum. The product is also easily dried, since it does not occlude solvent into the crystalline structure of the same.

Thus, the physical properties of the polymorph I of azelastine allow for good manipulation during all the preparation process. Furthermore, the process is reproducible and robust, and, therefore, easily industrializable.

The new crystalline form of the present invention can be converted into a pharmaceutically acceptable salt by methods known in the art, for instance, by reaction with the corresponding pharmaceutically acceptable acid in an appropriate solvent or by ions exchange between a salt of azelastine and an inorganic or organic salt. Preferably, the salt is the hydrochloride salt.

In a preferred embodiment, the preparation process of the polymorph I of azelastine of the present invention from the starting solution of azelastine in isobutylmethylketone further comprises a previous purification process which comprises carrying out a specific set of selective solvent extractions of azelastine or its impurities, said set of solvent extractions comprising: (a) carrying out at least one wash of an organic phase containing crude azelastine with water, at a pH equal or greater than 13 and at a temperature comprised between 40 °C and 80 °C, and separating the aqueous phase; (b) carrying out at least an extraction of the azelastine from the organic phase of step (a) with an aqueous solution of an acid at a pH comprised between 0.5 and 2 and at a temperature comprised between 50 and 80 °C; and (c) carrying out an extraction of the purified azelastine salt from the aqueous phase of step (b) with isobutylmethylketone at a pH comprised between 6.5 and 8.0 and at a temperature comprised between 20 °C and 70 °C, and separating the organic phase containing the azelastine. This purification process allows to purify some specific impurities derived of the process for the preparation of azelastine employed which are difficult to purify by other known methods. Among the impurities that may be effectively removed with the purification process of the present invention is the following one

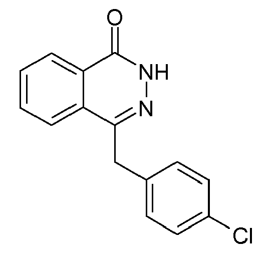

The set of extractions of steps (a) and (b) can be carried out with a solvent in which the azelastine is soluble. Preferably the solvent is an aliphatic ketone. Appropriate aliphatic ketones include methylpropylketone, isobutylmethylketone, ethylpropylketone, butylmethylketone, diisopropylketone, cyclopentanone and cyclohexanone. These ketones can also be used for carrying out the last extraction step (c), and as solvent for the subsequent crystallization of the crystalline form of the present invention.

Generally, the set of solvent extractions comprises: (a) one wash of an organic phase containing crude azelastine with water, at a pH equal or greater than 13; (b) one extraction of the azelastine from the organic phase of step (a) with an aqueous solution of an acid at a pH comprised between 0.5 and 2; and (c) one extraction of the purified azelastine salt from the aqueous phase of step (b) with isobutylmethylketone at a pH comprised between 6.5 and 8.0.

The most adequate conditions for carrying out said processes vary depending on the parameters considered by the expert in the art, such as, for example, the concentration of the starting material, temperature, and the like. These can be easily determined by said skilled person in the art by routine tests and with the help of the teachings of the examples given in this description.

The pharmaceutical composition of the present invention comprises a therapeutically effective amount of the polymorph I of azelastine of the present invention, together with suitable pharmaceutically acceptable excipients or carriers. The compound of the present invention can be normally formulated in accordance with standard pharmaceutical practice.

The polymorph I of azelastine of the present invention is useful in the treatment and/or prophylaxis of allergic diseases.

Throughout the description and the claims the word "comprises" and its variants are not meant to exclude other technical characteristics, additives, components or steps. For skilled persons in the art, other objects, advantages and characteristics of the invention can be deduced in part from the description and partly from the practice of the invention. The following examples are provided for illustrative means, and are not meant to be limiting of the present invention.

### EXAMPLES

### Example 1: Preparation of 1H-azepine, 4-hydrazinohexahydro-1-methyl, hydrochloride

208 g of benzoyl hydrazine (1.0 eq), 9.4 ml of water, and 174 g of potassium hydroxide 50% (1.00 eq) were mixed. Then, a solution of 250 g of 4H-azepine-4-one, hexahydro-1-methyl hydrochloride in 415 ml of water was added at room temperature. A solution of 43.35 g of sodium borohydride (0.75 eq.) in 312 ml of water was also added. Once the reaction was completed 1200 ml of ethyl acetate were added. A two layer solution was formed. After 20 minutes of stirring at room temperature, the organic layer was separated. The aqueous solution was extracted with 575 ml of ethyl acetate. The organic layers were combined and the solution was filtered off. Then, 204 g hydrochloric acid 36% (1.3 eq) at room temperature were added. A two layer solution was formed. The outcome aqueous solution was diluted with 250 ml of water, it was heated at reflux temperature and distilled until the temperature of the mixture was over 100 °C. Then, 400 g of hydrochloric acid were added (36%) (3 eq) and the mixture refluxed during 4 hours. The mixture was cooled down to 60 °C and 925 ml of xylene were added. The organic layer was separated and washed once with 465 ml of xylene at the same temperature. The aqueous phase was kept as a solution of 1H-azepine, 4-hydrazinohexahydro-1-methyl dihydrochloride.

### Example 2: Preparation of anhydrous azelastine, polymorph I

The aqueous solution of 1H-azepine, 4-hydrazinohexahydro-1-methyl dihydrochloride of the previous step, was cooled down to a temperature below 10 °C and 420 g of sodium hydroxide 50% (3.35 eq) were added. 420 g of benzoic acid, 2-[(4-chlorophenyl)acetyl] (1.0 eq.) and 1664 ml methanol were also added. The mixture was refluxed during 4 hours. Subsequently, it was cooled to 60 °C and 143 g of sodium hydroxide 50% (1.14 eq) were added. The reaction was heated and distilled until a temperature of 100 °C to remove the methanol.

The mixture was cooled down to 60 °C and 2050 ml of isobutylmethylketone (MIBK) and 500 ml of water were added. The aqueous layer was separated. 1750 ml of water and 172 g HCI (36%)(1.1 eq.) were added to the organic layer. The aqueous layer was separated. The organic layer was extracted at the same temperature with 525 ml of water. Some impurities of the process were dissolved in the organic layer. The aqueous layers were combined and 1880 ml of MIBK and 151 g of sodium hydroxide 50% (1.2 eq.) were added. The mixture was maintained 15 min. under stirring and the aqueous layer was separated. The organic layer was treated with charcoal. The treated solution was cooled down to 0-5 °C crystallizing a solid. After two hours at 0-5 °C, an off solid was filtered-off and washed with MIBK. The wet product was dried under vacuum to yield anhydrous azelastine. Purity by HPLC: 99.8% ¹H-RMN (dmso-⁶d, 400 MHz): 1.59-1.65 (1 H, m), 1.76-2.05 (5H, m), 2.24 (3H, s), 2.44-2.60 (5H, m), 4.32 (2H, s), 5.13-5.21 (1 H, m), 7.35 (4H, s), 7.80 (1 H, td, 7.2 Hz, 1.2 Hz), 7.86 (1 H, td, 8.0 Hz, 1.6 Hz), 7.95 (1 H, d, 8.0 Hz), 8.27 (1 H, dd, /.2 Hz, 1.2 Hz). ¹³C-RMN (dmso-⁶d, 100 MHz): 24.8 (t), 32.0 (t), 33.0 (t), 37.1 (t), 46.8 (q), 53.6 (t), 55.3 (d), 58.3 (t), 125.2 (d), 126.6 (d), 127.3 (s), 128.0 (s), 128.4 (d), 130.6 (d), 131.1 (s), 133.2 (d), 137.0 (s), 144.5 (s), 157.2 (s). IR (KBr): 3441, 2962, 2931, 2846, 2789, 1636, 1588, 1493, 1350, 1334, 775. mp: 113.0-114.1°C. KF: <0.1 %. The X-ray diffraction analysis gave the diffractogram shown in FIG. 1. The Differential Scanning Calorimetry analysis gave the curve shown in FIG. 2.

### Comparative Example: Preparation of azelastine monohydrate

A three-neck flask was charged with 25 g of anhydrous azelastine and 150 ml of a mixture of ethanol/water (1/1) were added. The reaction mixture was stirred for 10 minutes at room temperature, and then heated until dissolution (T^{a} 76 °C). The solution was slowly cooled and the product crystallized at 54 °C. The suspension was cooled at 0-5 °C and maintained for over 2 hours at this temperature. The solid product was isolated by filtration and washed with 5 ml of ethanol/water (1/1). The solid was dried at room temperature under vacuum to obtain 24 g of azelastine monohydrate as a white product. IR (KBr): 3526, 3396, 2929, 2792, 1635, 1586, 1490, 1340, 1324, 1015, 772. KF: 3.8% (theoretic for the monohydrate 4.5%). The X-ray diffraction analysis gave the diffractogram shown in FIG. 3. The Differential Scanning Calorimetry analysis gave the curve shown in FIG. 4.

## Claims

1. Crystalline form of azelastine of formula (I).

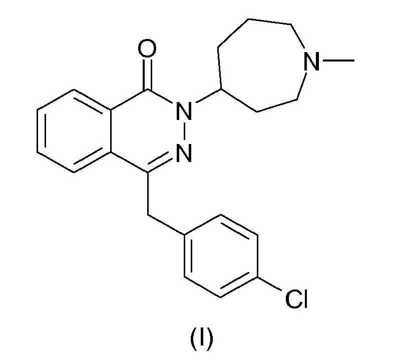

2. Polymorph I of azelastine of formula (I) **characterized by** having an X-ray diffractogram that comprises characteristic peaks at approximately 7.1, 10.7, 12.1, 13.5, 13.8, 16.8, 20.1, 20.8, 21.4 and 23.8 degrees 2 theta.

3. Polymorph I of azelastine according to claim 2, further **characterized by** an X-ray diffractogram as in FIG. 1.

4. A preparation process of the polymorph I of azelastine as defined in any of the claims 2-3, **characterized by** comprising the following steps:
a) crystallizing azelastine from a solution of said compound in isobutylmethylketone;
b) isolating the polymorph I of azelastine that appears in the prior step; and
c) removing the solvent from the polymorph I of azelastine thus obtained.

5. The preparation process according to claim 4, further comprising the conversion of the azelastine into a pharmaceutically acceptable salt by reaction of azelastine with a pharmaceutically acceptable acid, or alternatively, by reaction of a salt of azelastine with an organic or inorganic salt.

6. The preparation process according to any of the claims 4-5, further comprising a previous purification process which comprises carrying out a specific set of selective solvent extractions of azelastine or its impurities, said set of solvent extractions comprising:
(a) carrying out at least one wash of an organic phase containing crude azelastine with water, at a pH equal or greater than 13 and at a temperature comprised between 40 °C and 80 °C, and separating the aqueous phase;
(b) carrying out at least an extraction of the azelastine from the organic phase of step (a) with an aqueous solution of an acid at a pH comprised between 0.5 and 2 and at a temperature comprised between 50 and 80 °C; and
(c) carrying out an extraction of the purified azelastine salt from the aqueous phase of step (b) with isobutylmethylketone at a pH comprised between 6.5 and 8.0 and at a temperature comprised between 20 °C and 70 °C, and separating the organic phase containing the azelastine, which is the starting solution of said compound in isobutylmethylketone of claim 4 step a);

7. A pharmaceutical composition comprising a crystalline form of azelastine as defined in any of the claims 1-3, together with appropriate amounts of pharmaceutical excipients or carriers.

8. Use of a crystalline form of azelastine as defined in any of the claims 1-3 for the preparation of a medicament for the treatment and/or prophylaxis of allergic diseases.

9. Crystalline form of azelastine as defined in any of the claims 1-3, for use in the treatment and/or prophylaxis of allergic diseases.
